(19) Europäisches Patentamt
European Patent Office
Office européen des brevets

(11) **EP 2 484 389 A1**

(12) **EUROPEAN PATENT APPLICATION**

(43) Date of publication:
08.08.2012 Bulletin 2012/32

(51) Int Cl.:
*A61L 27/44* (2006.01)     *A61L 27/46* (2006.01)
*A61L 27/58* (2006.01)     *A61L 27/18* (2006.01)
*A61L 27/32* (2006.01)

(21) Application number: 12153887.0

(22) Date of filing: 03.02.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 03.02.2011 JP 2011021790

(71) Applicants:
• Orthorebirth Co., Ltd.
Kanagawa 224-0033 (JP)
• Nagoya Institute of Technology
29, Aza Kiichi
Gokiso-cho
Showa-ku
Nagoya-shi
Aichi 466-8555 (JP)
• Yabashi Industries Co., Ltd.
Ogaki City Gifu 5032213 (JP)
• Showa Ika Kohgyo Co., Ltd.
Toyohashi-shi
Aichi 441-8026 (JP)

(72) Inventors:
• KASUGA, Toshihiro
Nagoya-shi, Aichi 466-8555 (JP)
• OTA, Yoshio
Yokohama-shi, Kanagawa 224-0033 (JP)
• YAO, Xianfeng
Yokohama-shi, Kanagawa 224-0033 (JP)

(74) Representative: Giovannini, Francesca et al
Osha Liang
32, avenue de l'Opéra
75002 Paris (FR)

(54) **Biodegradable fiber and fiber wadding for filling bone defects and method for producing the same**

(57) A fiber wadding formed from a biodegradable polymer fiber containing calcium carbonate fine particles including silicon that is **characterized in that** hydroxyapatite is precipitated and scattered nearly uniformly on the surface of the biodegradable polymer fiber is disclosed. Further, a method for production of fiber wadding that includes the steps of heating and kneading silicon-containing calcium carbonate fine particles and a biodegradable polymer to produce a composite, dissolving the composite by mixing the composite with a solvent to obtain a spinning solution, processing the spinning solution into fiber wadding by using electrospinning method, and alternatingly immersing the fiber wadding in a calcium aqueous solution and a phosphate aqueous solution to cause precipitation of hydroxyapatite in an approximately uniformly scattered manner on the surface of the fibers is disclosed.

Fig. 6

EP 2 484 389 A1

**Description**

[Field of the Invention]

The present invention relates to a biodegradable polymer fiber and a fiber wadding produced from such biodegradable fiber that are used as a bone filling material for medical treatment of bone defects resulted from external injury (i.e. broken bones or the like), removal of tumors, spinal surgery, artificial joints, or the like.

[Background Art]

Maintenance of exercise capacity is extremely important for heath preservation in a rapidly aging society, and an immediate cure is needed for bone defects. Vast majority of artificial bone presently used are in the forms of blocks, granules, or pastes. In the case of ceramic such as calcium phosphate that is already on the market, the ceramic is not readily processed into the various types of bone shapes of humans. Moreover, when the bone defect is filled by a granular or paste type material, there is accompanying danger of leakage depending on the condition of the defective part. In order to perform better medical treatment of bone defects, a preferred material would be a fibrous material that has a function for causing acceleration of bone regeneration and is gradually absorbed and replaced by bone. If the material is in the form of a flexible wadding, the material would simply and reliably fill even a large defect in a very short time in a manner that fits the defect well and without falling out, and such a material would assure sufficient space for intrusion of bone.

Several fibrous structures of biodegradable polymers and production methods for the same have been proposed. For example, there is a fibrous processed material obtained by using centrifugal force to extrude melted polylactic acid from a fine gap, followed by cooling in air (Japanese Unexamined Laid-open Patent Application No. H7-268751), a composite type filler material characterized by combination of a fibrous bioabsorbable organic material and a calcium phosphate compound (Japanese Unexamined Laid-open Patent Application No. 2000-262608), a polymer fiber sponge-shaped three dimensional structured body produced using the electrospinning method (Japanese Unexamined Laid-open Patent Application No. 2008-261064), or the like. Moreover, fibrous structured bodies have been proposed that include a drug in a biodegradable polymer fiber capable of medical treatment by in vivo release (Japanese Unexamined Laid-open Patent Application No. 2003-506401, Japanese Unexamined Laid-open Patent Application No. 2009-261448). However, presently no material has been reported that has excellent bioabsorption ability and bone-forming ability, and is also capable of securing space for invasion by bone tissue using a fiber-like three-dimensional structure.

Presently, although there are numerous reports of attempts to include DNA or bone-forming protein in a bioabsorbable material in order to improve bone forming ability, they are extremely expensive, handling is difficult, and sustaining a long-term effect is difficult. On the other hand, it has been reported that an extremely minute amount of the silicon ion promotes bone formation by genetic stimulation of osteoblastic cells (I. D. Xynos, et al, Biochem. Biophys. Res. Comm., 276, 461 (2000)). An inexpensive long-term effect can be expected if this type of inorganic factor can be utilized. When a composite of calcium carbonate (vaterite) and polylactic acid (PLA) is immersed in a simulated biological fluid (SBF), it has been shown that bone-like apitite grows on the surface of the composite in a short period of time (H. Maeda, et al , J. Ceram. Soc. Japan, 112, S804 (2004)), and an nonwoven fabric formed from silicon-eluting type calcium carbonate and PLA has been shown to have the ability to form bone in animal experiments (A. Obata, et al, Acta Biomaterialia, 6, 1248 (2010)). Based on these facts, the combination of silicon, vaterite, and PLA to form a fibrous material is thought to be an important means for providing an ideal bone filling material that has excellent bone-forming function and bioabsorbance and meets clinical requirements. The disclosures of such earlier literatures are incorporated by reference in the disclosure of the present application.

[Problem to Be Solved by the Invention]

One aspect of the present invention is to provide a bioabsorbable bone filling material and a production method thereof that has a bone formation promotion function and wide range of applicability in surgery and is capable of reducing the time required for surgery.

[Means for Solving the Problem]

One aspect of the present invention is a biodegradable polymer fiber that includes silicon-eluting type calcium carbonate. It provides a controlled silicon and calcium releasing fiber having hydroxyapatite precipitated on a surface of the biodegradable polymer fiber. It also provides a fiber wadding formed from such type of fibers.

According to an aspect of the present invention, the silicon-eluting type calcium carbonate is vaterite phase calcium carbonate containing silicon. The silicon-eluting type calcium carbonate is dispersed in the biodegradable polymer in the form of fine particles, and a composite is formed from the silicon-eluting type calcium carbonate and the biodegradable polymer.

According to an aspect of the present invention, the surface of the silicon-eluting type calcium carbonate-containing biodegradable polymer fiber is coated by hydroxyapatite in a nearly uniformly scattered manner, wherein the surface of the fiber is not completely covered by hydroxyapatite.

According to an aspect of the present invention, when the fiber wadding of the present invention is filled in a bone defect part, silicon containing ions such as ($HSiO_3^-$) that are necessary for bone formation (hereinafter referred to as silicon-type ions) and calcium ions can be effectively supplied from a surface of the fiber, and early adhesion of the osteoblasts to the fiber wadding by the hydroxyapatite can be secured.

According to an aspect of the present invention, such type of fiber and fiber wadding can be produced by a method that comprises the steps of:

(1) producing a composite of silicon-eluting type calcium carbonate and a biodegradable polymer;
(2) preparing a spinning solution by dissolving the composite by using a solvent and then forming fiber wadding from the biodegradable polymer fiber by using the electrospinning method; and
(3) alternatingly immersing the fiber wadding in a calcium aqueous solution and a phosphoric acid aqueous solution to cause precipitation of hydroxyapatite on the surface of the fibers in an approximately uniformly scattered manner.

According to an aspect of the present invention, by using an alternating immersion method that alternatingly immerses the fiber wadding obtained by electrospinning in a calcium aqueous solution and a phosphate aqueous solution, it becomes possible to cause precipitation of hydroxyapatite on the surface of the fibers in a short period of time. Thus elution of silicon from the calcium carbonate fine particles that are dispersed in the fibers during the process of precipitation of hydroxyapatite on the fiber surface can be suppressed.

[Advantage of the Invention]

The biodegradable polymer fiber of the present invention and the fiber wadding formed from such fiber, when filled in a bone defect part, are capable of stably maintain a high bone-forming effect due to gradual release of silicon-type ions and calcium ions. Moreover, because the fiber wadding formed of such fibers is flexible, the fiber wadding can be easily filled in bone defect parts of various shapes. It is also expected that this fiber wadding can be used as a bioabsorbable bone filling material that is resistant to falling out. Using the production method of the fiber wadding according to the present invention, it is possible to readily produce a bone-filling material that is convenient to use and has an excellent bone-formation promotion function as explained above.

[Brief Description of the Drawings]

[FIG 1] FIG 1 is an SEM image of a fine particles of silicon-eluting type calcium carbonate used in Example 1.
[FIG 2] FIG 2 is an x-ray diffraction pattern of the silicon-eluting type calcium carbonate used in Example 1.
[FIG 3] FIG 3 is a drawing for explanation of the electrospinning method used in the examples of the present invention.
[FIG 4] FIG 4 is an SEM image of a fiber cross section of the fiber wadding produced in Example 1.
[FIG 5] FIG 5 is an photograph showing appearance of sample 1 produced in Example 1.
[FIG 6] FIG 6 is an SEM image of the surface of the fiber of sample 1 produced in Example 1.
[FIG 7] FIG 7 shows the IR absorption spectra of samples 1, 3, and 4.
[FIG 8] FIG 8 shows characteristics of elution of Si and Ca of sample 1 produced in Example 1 into tris buffer solution.
[FIG 9] FIG 9 shows characteristics of elution of Si and Ca into tris buffer solution of sample 2 produced in Example 2.
[FIG 10] FIG 10 is an SEM image of the surface of the fiber of sample 3 produced in Comparative Example 1.
[FIG 11] FIG 11 shows characteristics of elution of Si and Ca of sample 3 produced in Comparable Example 1 into tris buffer solution.
[FIG 12] FIG 12 is an SEM image of the surface of the fiber of sample 4 produced in Comparative Example 2.
[FIG 13] FIG 13 shows characteristics of elution of Si and Ca of sample 4 produced in Comparable Example 2 into tris buffer solution.

[Detailed Description of the Invention]

[Silicon-eluting type Calcium Carbonate]

Vaterite phase calcium carbonate may be preferably used as the silicon-eluting type calcium carbonate for the production of the controlled silicon and calcium releasing fiber of the present invention.

Among the calcite, aragonite, and vaterite crystal phases of calcium carbonate, the reason why vaterite phase is preferably used is that silicon can be easily introduced into the vaterite phase, vaterite phase has the highest solubility in water, and that hydroxyapatite can be easily generated on the surface of a composite formed from the vaterite phase and polylactic acid.
As disclosed in the Japanese Unexamined Laid-open Patent Application No. 2008-100878, this type of silicon-eluting type calcium carbonate can be obtained in the form of fine particles by a carbonation process that injects carbon dioxide gas into a suspension obtained by mixing methanol, water, hydrated lime, and an organic silicon compound.

The content of silicon in the aforementioned vaterite phase calcium carbonate (fine particles) can be adjusted according to the type and added amount of the organic silicon compound used during the aforementioned process. In the examples of the present invention, $\gamma$-aminopropyl triethoxysilane (APTES, $C_9H_{23}NO_3Si$) was used as the silicon source. The utilized Si content in the calcium carbonate is preferably 0.5 to 5 wt%, and further preferably is about 2 wt%.

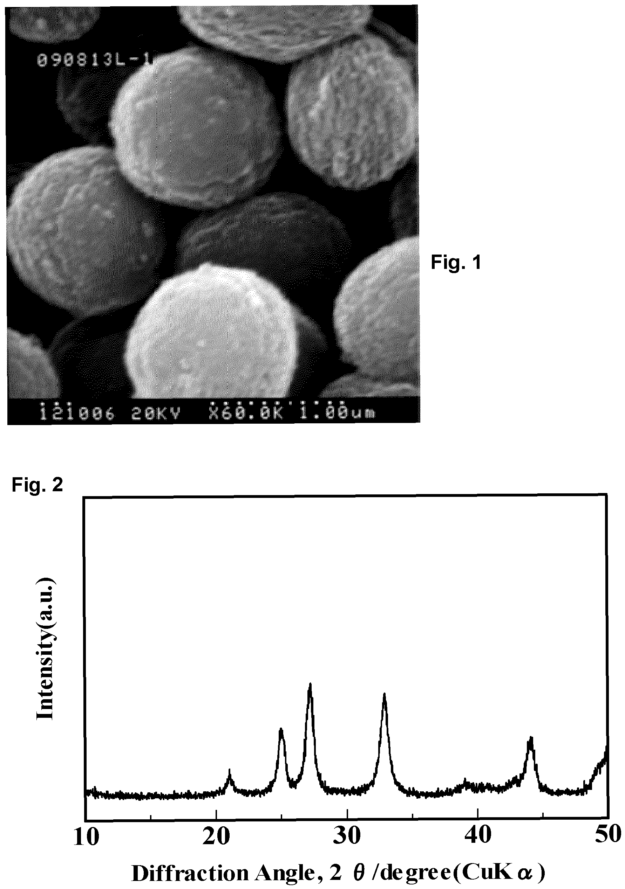

FIG 1 is a scanning electron microscope image (SEM image) of silicon-eluting type calcium carbonate obtained by the aforementioned method. It is understood that the fine particles of vaterite generated by the reaction with APTES was agglomerated to form spherical particles of about 1 $\mu$m diameter. It's specific surface area was 67 $m^2$/g. It was confirmed from the x-ray diffraction (XRD) pattern shown in FIG 2 that the obtained silicon-eluting type calcium carbonate was in vaterite phase.

[Biodegradable Polymer]

The biodegradable polymer used for the production of the controlled silicon and calcium releasing fiber of the present invention is preferably polylactic acid or a copolymer of polylactic acid and polyglycolic acid. The utilized biodegradable polymer preferably has a molecular weight of 150,000 to 300,000.

[Composite]

A heating kneader is used for heating and kneading the silicon-eluting type calcium carbonate fine particles and polylactic acid to produce a composite of silicon-eluting type calcium carbonate and polylactic acid. The content of the silicon-eluting type calcium carbonate in the composite is preferably 20 to 40 wt%, most preferably about 30 wt% in consideration of Si and Ca elution characteristics and fiber mechanical characteristics (i.e. strength and elasticity) when the spinning solution of the composite is made into fibers by electrospinning.

[Fiber wadding]

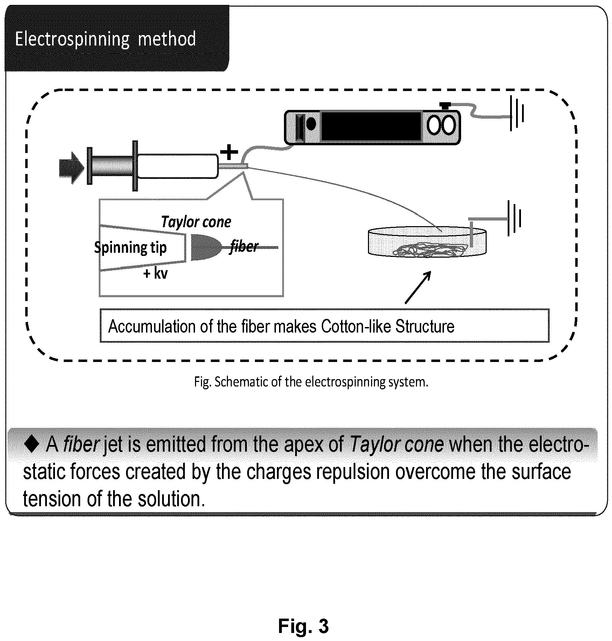

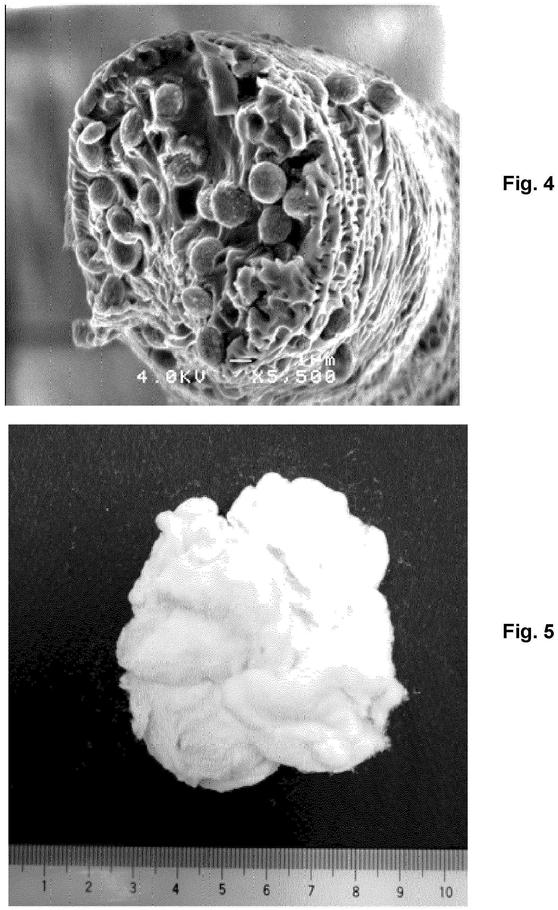

The composite of polylactic acid and silicon-eluting type calcium carbonate fine particles (silicon-eluting type calcium carbonate content = 30 wt%) is dissolved by a solvent such as chloroform ($CHCl_3$) at a concentration of 7 to 10 wt% (i.e. [polylactic acid (weight amount)/(chloroform + polylactic acid (weight amounts))] = 0.07 to 0.10), more preferably at a concentration of 7 to 8 wt%, to prepare the spinning solution.
The obtained spinning solution was processed into fibers by the electrospinning method. In the electrospinning method, a high positive voltage is applied to the spinning solution, solvent is volatized in the electrical field, fibers produced are directed toward grounded ethanol within a vessel to form fiber wadding in the ethanol. In conducting such spinning, it is possible to produce fibers and fiber wadding of the desired form by adjusting spinning conditions, i.e. concentration of the spinning solution, type of solvent, feed rate, spinning time, applied voltage, distance between the nozzle and ethanol, or the like. FIG 3 shows a general description of the electrospinning method used in the present embodiment. Diameter of the fiber obtained by the aforementioned method is about 10 to 20 $\mu$m. As shown in FIG 4, silicon-eluting type calcium carbonate fine particles are dispersed in the polylactic acid (PLA) matrix resin of these fibers.

[HAp Coating]

The fiber wadding produced by the aforementioned method was collected from the ethanol, dried, and made hydrophilic using ethanol.
Thereafter, the fiber wadding having undergone the above described treatment was immersed in a calcium aqueous solution. Thereafter, the fiber wadding was immersed in phosphate aqueous solution, thereby causing uniformly scattered precipitation of hydroxyapatite on the surface of the fibers. A calcium chloride ($CaCl_2$) aqueous solution was used as the calcium aqueous solution, and sodium dihydrogen phosphate ($Na_2HPO_4$) was used as the phosphate aqueous solution.

Making HAp coating on the fiber comes from the report that cell adhesion of the fibers is improved by HAp coating because of increase of hydrophilicity of the fibers in comparison to the fibers without having HAp coating.

The mechanism of the precipitation of hydroxyapatite on a surface of the fibers by the aforementioned treatment is not necessarily clear. However, according to the knowledge of the inventors of the present invention, immersion of the fibers in the calcium aqueous solution causes Ca ions to be present on the surface, and by immersing the fiber in the phosphate aqueous solution in the next step, $PO^4$ ions are supplied, and apatite is generated on the fiber surface. The fiber wadding having HAp precipitated on the fiber surface by the aforementioned alternating immersion method is water washed and then dried at 50°C. Preferably, after immersion in ethanol, the fibers are left to dry naturally (room temperature draft), or alternatively, the fibers are vacuum dried (room temperature).

[Examples]

(Example 1)

Examples of the production method of fiber wadding of the present invention will be explained below. The below listed examples are for deeper understanding of the present invention, and the present invention is not limited in any respect to the below listed examples.

1. Utilized Material:

In Example 1, the below listed materials were used for producing fine particles of vaterite phase calcium carbonate that contains 2 wt% of Si (2SiV).

[Vaterite Phase Calcium Carbonate (2SiV)]

Hydrated lime (at least 95 percent purity, manufactured by Yabashi Industries Co., Ltd.), methanol (special reagent grate, 99.8 percent or better purity, manufactured by Kishida Chemical Co., Ltd.), γ-aminopropyl triethoxysilane (TSL8331, at least 98% purity, Momentive Performance Materials Japan, Inc.), carbon dioxide gas (high purity liquefied carbon dioxide gas, 99.9 percent purity, Taiyo Kagaku Kogyo Co., Ltd.).

2.0 L of methanol, 0.1 L of distilled water, 150 g of hydrated lime, and 60 mL of tetraethoxysilane were mixed. While this mixture was stirred at 20°C using a turbine blade agitator, carbon dioxide gas (2 L/minute) was introduced for 75 minutes to cause gel formation. After the gel broke down, the suspension was passed through a 53 μm size opening sieve. Then the product was recovered by filtration and was dried to obtain sample 1 (2SiV).

[Polylactic Acid (PLA)]

Poly (L-lactide) of a molecular weight of 200,000 to 300,000 (manufactured by PURAC Biochem BV) was used as the polylactic acid (PLA) in Example 1.

2. Preparation of the Composite of Biodegradable Polymer and Silicon-eluting type Calcium Carbonate:

42 g of PLA and 18 g of 2SiV (fine particles) were kneaded for 45 minutes at 200°C using a heating kneader to produce the composite of PLA and 2SiV (2SiV content = 30 percent by weight).

3. Preparation of the Spinning Solution:

$CHCl^3$ was mixed with the composite at a ratio of 9.3 g of $CHCl_3$ per 1 g of the composite, and the mixture was dissolved to obtain the spinning solution. Chloroform ($CHCl_3$, special reagent grade, at least 99.0 percent purity, manufactured by Kishida Chemical Co., Ltd.) was used as the solvent for dissolving the composite.

4. Preparation of Fiber and Fiber Wadding by Electrospinning

The spinning solution obtained in the above described manner was used to produce fiber wadding by the electrospinning method.

The spinning solution feed rate was 0.2 mL/minute, the applied voltage was 17 kV, the distance between the nozzle and the collector (ethanol placed in the vessel) was about 20 cm, the injection nozzle was an 18G type, and special reagent grade ethanol (purity of at least 99.5 percent, manufactured by Kishida Chemical Co., Ltd.) was used.

FIG 5 shows an external view of the sample 1 obtained in the aforementioned manner. Sample 1 can be confirmed to be fiber wadding. FIG 4 shows a SEM image of a cross section of the fiber of the fiber wadding of sample 1. 2SiV spherical particles were observed to be dispersed within the polylactic acid of the fibers.

5. HAp Coating

The produced fiber wadding was alternatingly immersed in the calcium aqueous solution and in the phosphate aqueous solution to cause precipitation of fine hydroxyapatite uniformly scattered on a surface of the fibers.

Procedure: (Alternating Immersion Method)

(1) 0.05 g of the fiber wadding was immersed in 10 mL of ethanol to make the fiber wadding hydrophilic. Thereafter, the fiber wadding was washed using purified water, and then the fiber wadding was immersed for about 10 seconds in 50 mL of room temperature $CaCl_2$ aqueous solution (concentration = 0.2 mol/L). Then the fiber wadding was washed using ultrapure water.

(2) Thereafter, the fiber wadding was immersed for 1 hour in 50 mL of $Na_2HPO_4$ at 37˚C. Then the fiber wadding was washed using ultrapure water and then was dried at 50˚C.

For the Calcium Chloride, Anhydrous $CaCl_2$ (special reagent grade, purity greater than or equal to 95.0 percent, manufactured by Kishida Chemical Co., Ltd.) was used.

For the Di sodium Hydrogen Phosphate, Disodium hydrogen phosphate (anhydrous, $Na_2HPO_4$, special reagent grade, purity greater than or equal to 99.0 percent, manufactured by Kishida Chemical Co., Ltd.) was used.

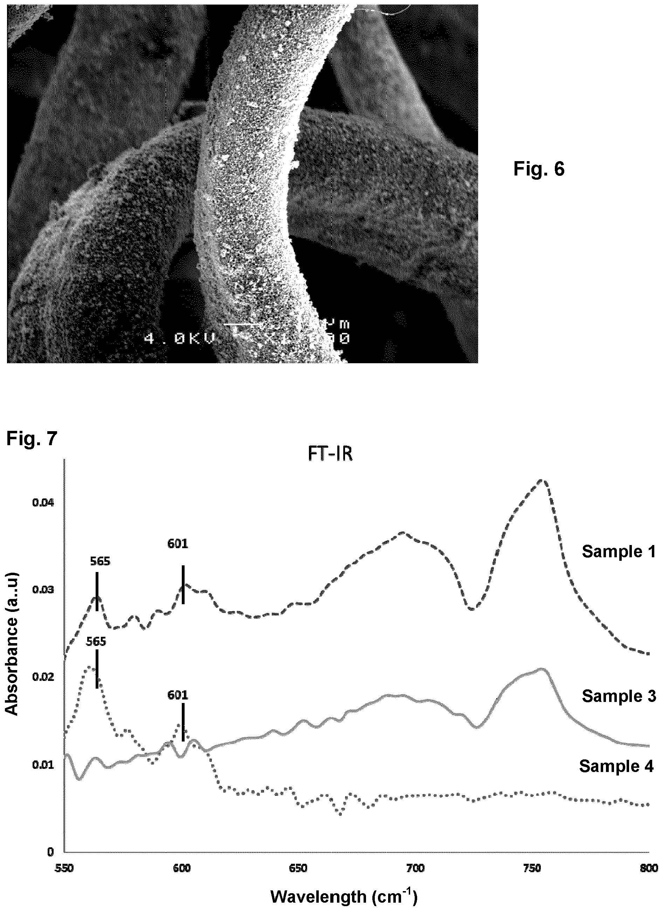

A SEM image of the fiber surface is shown in FIG 6. Fine white precipitates scattered nearly uniformly on the surface of the fiber are observed. FIG 7 shows the IR absorption spectrum of sample 1. Absorption peaks (565 and 601 cm$^{-1}$) were found indicating the phosphate group ($PO_4$) and suggesting that the precipitate is hydroxyapatite. The amount of hydroxyapatite precipitate is estimated to be 1.6 wt%.

Sample 1 was thermally decomposed and dissolved, and the resultant solution was analyzed quantitatively for P(phosphorous) using ICP. Quantity of P was found to be 0.29 wt%. From the difference in the amount of P versus the amount of P found in sample 3 (0.002 wt% assumed to be contained in the 2SiV) the amount of hydroxyapatite ($Ca_{10}(PO_4)_6(OH)_2$, 1004.62) precipitated on the fiber of sample 1 was calculated.

$$Ca_{10}(PO_4)_6(OH)_2 / 6P = 1004.62 / 6 \times 30.97 \fallingdotseq 5.4$$

$$(0.29 - 0.002) \times 5.4 \fallingdotseq 1.6$$

6. Evaluation of Silicon and Calcium Release Ability of the Obtained Fiber / Fiber Wadding. From the fiber/fiber wadding obtained in the aforementioned manner, release of silicon and calcium was observed using a scanning electron microscope (SEM) TSM-6301F (JEOL Ltd.). An infrared (IR) absorption spectrum, which was obtained using a Fourier transform infrared spectrometer (FTIR) FT/IR-4100 (JASCO Corp.), was used for identification of the precipitate. Si content of the calcium carbonate was determined by an x-ray fluorescence spectrometer (FX) ZSX Primus II (Rigaku Corp.), and the concentrations of Si and Ca in the tris buffer solution were measured using a high frequency plasma emission spectrometer (ICP) ICPS-7510 (Shimadzu Corp.). The electrospinning apparatus was self-manufactured using a high voltage power supply (High Voltage Power Supplier, HARB-40P0.75, Matsuda Precision, Inc.) and a syringe pump (Infusion Pump model FP-W-100, Melquest Ltd.). A 0.05 mol/L tris-HCl buffer solution (pH = 7.6), manufactured by Wako Pure Chemical Industries, Ltd. was used as the tris buffer solution.

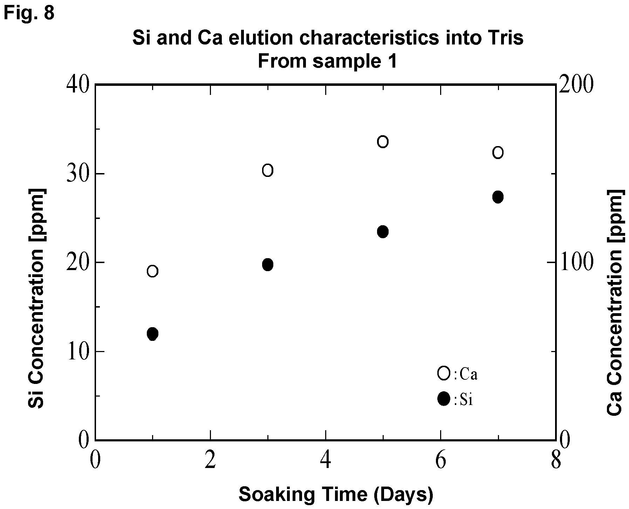

0.25 g of sample 1 was immersed in 50 mL of the tris buffer solution, and the buffer solution was left to stand in an isothermal chamber maintained at 37˚C. After immersion for a certain period of time, solids and liquid were separated, and the concentrations of Si and Ca in the liquid were measured using ICP. The respective elution characteristics of Si and Ca into tris buffer solution are shown in FIG 8. A tendency that Si ions and Ca ions are both gradually eluted was observed.

(Example 2)

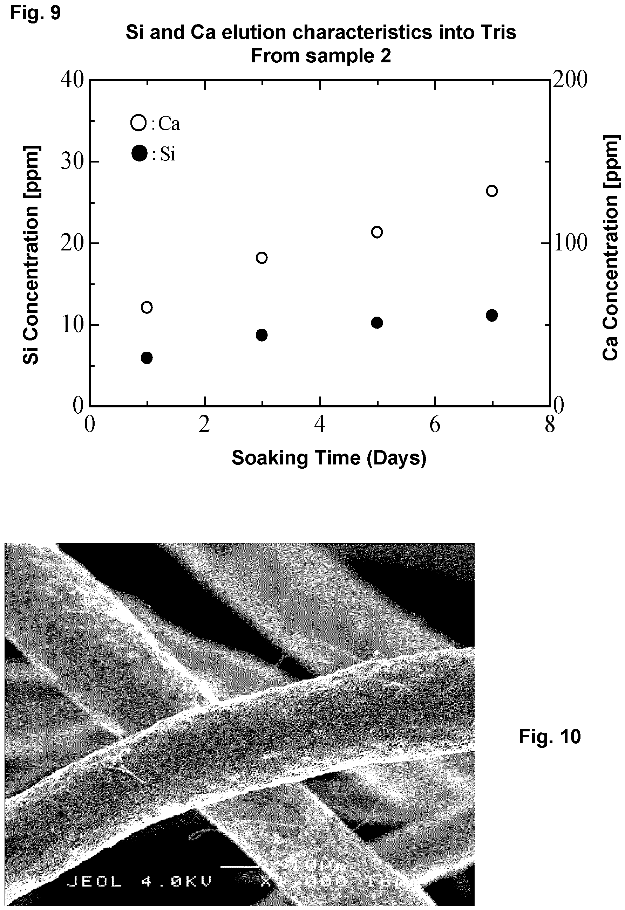

After production of a fiber wadding under the same manufacturing conditions as those of Example 1, only the immersion time for emersion of the fiber wadding in 50 mL of the 37˚C disodium hydrogen phosphate (anhydrous, $Na_2HPO_4$, concentration = 0.12 mol/L) solution was changed to be four hours in the alternating immersion method. Then, after washing using ultrapure water, the fiber wadding was dried at 50˚C.
Result of measurement of elution of Si and Ca, measured in the same manner as in Example 1 is shown in FIG 9 for the obtained sample 2. Although a tendency that both Si and Ca are gradually eluted is observed, tendency of the controlled release of Si ions is weaker than in Example 1.

(Comparative Example 1)

FIG 10 shows an SEM image of the surface of the fibers of sample 3 which was obtained under the same manufacturing conditions as those of Example 1 without subjecting it to the alternating immersion. A precipitate was not observed on the porous polylactic acid surface, and no absorption peak for the phosphate ($PO_4$) group was found in FIG 7. P in the solution which was obtained by having the sample 3 thermally decomposed and dissolved was analyzed quantitatively by using ICP and it was found that P concentration was 0.002 wt%. It is assumed that the P was contained in the calcium carbonate (2SiV).
0.25 g of sample 3 was immersed in 50 mL of the tris buffer solution and was left to stand in an isothermal chamber maintained at 37˚C. After immersion for a certain period of time, solids and liquid were separated, and the concentrations of Si and Ca in the liquid were measured using ICP. The respective elution characteristics of Si and Ca into tris buffer solution are shown in FIG 10. A tendency was found that only Si ions are gradually eluted.

(Comparative Example 2)

After production of fiber wadding under the same manufacturing conditions as those of Example 1, the fiber wadding was immersed for 24 hours in 1.5-fold concentrated SBF (simulated body fluid) at 37˚C. Thereafter, the sample was washed using distilled water and dried at room temperature.

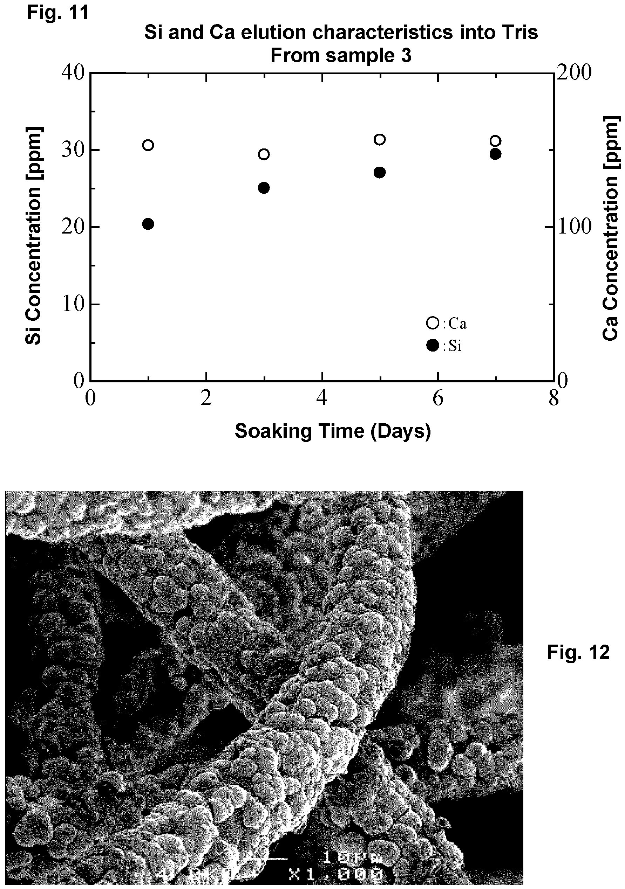

FIG 11 shows the surface of the fibers of the obtained sample 4. The surface was observed to be thoroughly coated by a precipitate. The IR absorption spectrum of sample 4 is shown in FIG 7. Absorption peaks (565 and 601 cm$^{-1}$) were found indicating the phosphate ($PO_4$) group and suggesting that the precipitate was hydroxyapatite. The estimated amount of precipitation of hydroxyapatite was 4.2 wt%. P in the solution which was obtained by having the sample 4 thermally decomposed and dissolved was analyzed quantitatively by using ICP and it was found that P concentration was 0.78 wt%. After subtracting the amount of P measured in sample 3 (0.002 wt%, assumed to be contained in the 2SiV), the amount of hydroxyapatite ($Ca_{10}(PO_4)_6(OH)_2$: 1004.62) precipitated on the fibers of sample 4 was calculated.

$$Ca_{10}(PO_4)_6(OH)_2 / 6P = 1004.62 / 6 \times 30.97 \fallingdotseq 5.4$$

$$(0.78 - 0.002) \times 5.4 \fallingdotseq 4.2$$

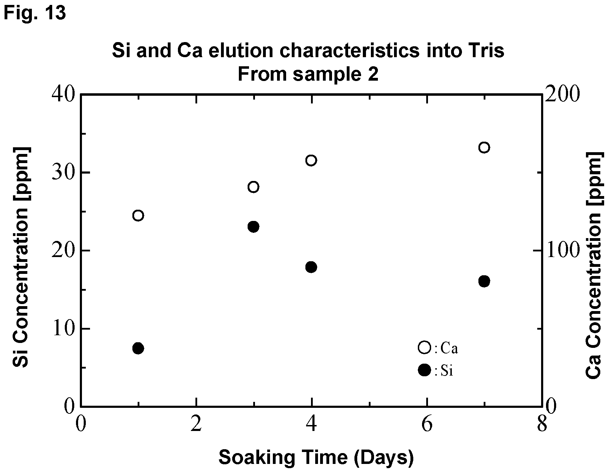

0.25 g of sample 4 was immersed in 50 mL of the tris buffer solution, and the buffer solution was left to stand in an isothermal chamber maintained at 37˚C. After immersion for a certain period of time, solids and liquid were separated, and the concentrations of Si and Ca in the liquid were measured using ICP. The respective elution characteristics of Si and Ca into tris buffer solution are shown in FIG 13. A tendency was found that only Ca ions are gradually eluted.
Although the present invention was explained using the aforementioned limited embodiments, one skilled in the art would understand the possibility of other embodiments based on disclosure of the present application. Such other embodiments do not depart for the scope of the present invention. Thus the scope of the present invention should be restricted to the scope according to the claims appended to the present specification.

**Claims**

1. A fiber wadding comprising a biodegradable polymer fiber that comprises calcium carbonate fine particles containing silicon, wherein hydroxyapatite is precipitated on a surface of the biodegradable polymer fiber.

**2.** The fiber wadding comprising a biodegradable polymer fiber according to claim 1, wherein the hydroxyapatite is approximately uniformly scattered on the surface of the biodegradable polymer fiber.

**3.** The fiber wadding comprising a biodegradable polymer fiber according to claim 1, wherein the biodegradable polymer is a polymer selected from the group consisting of: polylactic acid and copolymers of polylactic acid and polyglycolic acid.

**4.** The fiber wadding comprising a biodegradable polymer fiber according to claim 1, wherein the calcium carbonate is vaterite phase calcium carbonate.

**5.** A method for producing a fiber wadding comprising a biodegradable fiber, the method comprises the steps of:

heating and kneading silicon-containing calcium carbonate fine particles and a biodegradable polymer to produce a composite;
dissolving the composite by mixing the composite with a solvent to obtain a spinning solution and processing the spinning solution into fiber wadding by using electrospinning method; and
alternatingly immersing the fiber wadding in a calcium aqueous solution and a phosphate aqueous solution to cause precipitation of hydroxyapatite in an approximately uniformly scattered manner on a surface of the fibers.

**6.** The method for production of a fiber wadding comprising a biodegradable fiber according to claim 5, wherein the biodegradable polymer is a polymer selected from the group consisting of: polylactic acid and copolymers of polylactic acid and polyglycolic acid.

**7.** The method for production of a fiber wadding comprising a biodegradable fiber according to claim 5, wherein the silicon-containing calcium carbonate is vaterite phase calcium carbonate.

Fig. 1

Fig. 2

Intensity(a.u.)

Diffraction Angle, 2 $\theta$ /degree(CuK $\alpha$ )

Fig. Schematic of the electrospinning system.

◆ A *fiber* jet is emitted from the apex of *Taylor cone* when the electrostatic forces created by the charges repulsion overcome the surface tension of the solution.

**Fig. 3**

Fig. 4

Fig. 5

**Fig. 6**

**Fig. 7**

**Fig. 8**

Si and Ca elution characteristics into Tris
From sample 1

**Fig. 9**

## Si and Ca elution characteristics into Tris
## From sample 2

**Fig. 10**

**Fig. 11**

Si and Ca elution characteristics into Tris
From sample 3

**Fig. 12**

**Fig. 13**

Si and Ca elution characteristics into Tris
From sample 2

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/009522 A1 (KASUGA TOSHIHIRO [JP] ET AL) 13 January 2011 (2011-01-13) * paragraphs [0010], [0012], [0032], [0034], [0035]; claims; examples * ----- | 1-7 | INV.<br>A61L27/44<br>A61L27/46<br>A61L27/58<br>A61L27/18<br>A61L27/32 |
| X | OBATA A ET AL: "Electrospun microfiber meshes of silicon-doped vaterite/poly(lactic acid) hybrid for guided bone regeneration", ACTA BIOMATERIALIA, ELSEVIER, AMSTERDAM, NL, vol. 6, no. 4, 1 April 2010 (2010-04-01), pages 1248-1257, XP026921444, ISSN: 1742-7061, DOI: 10.1016/J.ACTBIO.2009.11.013 [retrieved on 2009-11-11] * the whole document * ----- | 1-7 | |

TECHNICAL FIELDS
SEARCHED      (IPC)

A61L

The present search report has been drawn up for all claims

| Place of search | Date of completion of the search | Examiner |
|---|---|---|
| The Hague | 28 June 2012 | Derrien, Anne-Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 15 3887

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2012

| Patent document cited in search report | Publication date | Patent family member(s) | Publication date |
|---|---|---|---|
| US 2011009522 A1 | 13-01-2011 | JP 2011015865 A<br>US 2011009522 A1 | 27-01-2011<br>13-01-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H7268751 B
- JP 2000262608 A
- JP 2008261064 A
- JP 2003506401 A
- JP 2009261448 A
- JP 2008100878 A

**Non-patent literature cited in the description**

- **I. D. XYNOS et al.** *Biochem. Biophys. Res. Comm.,* 2000, vol. 276, 461
- **H. MAEDA et al.** *J. Ceram. Soc. Japan,* 2004, vol. 112, S804
- **A. OBATA et al.** *Acta Biomaterialia,* 2010, vol. 6, 1248